# EUROPEAN PATENT SPECIFICATION

(11) **EP 2 611 906 B1**
(45) Date of publication and mention of the grant of the patent: **03.01.2024**
(21) Application number: 11822565.5
(22) Date of filing: 31.08.2011
(51) Int. Cl.: C12N 5/10, C12N 5/07, A61K 35/28, A61K 35/38, A61P 1/00, A61P 1/04, A61P 1/16, A61P 3/10, A61P 7/02, A61P 7/04, A61P 7/06, A61P 9/00, A61P 9/04, A61P 37/06, A61P 11/00, A61P 13/12, A61P 17/00, A61P 17/04, A61P 17/12, A61P 19/00

(54) **SYSTEMIC, ALLOGENIC STEM CELL THERAPIES FOR TREATMENT OF DISEASES IN CATS AND DOGS**
SYSTEMISCHE ALLOGENE STAMMZELLTHERAPIEN ZUR BEHANDLUNG VON KRANKHEITEN IM KATZEN UND HUNDEN
THÉRAPIES SYSTÉMIQUES PAR CELLULES SOUCHES ALLOGÉNIQUES POUR LE TRAITEMENT DES MALADIES CHEZ LES CHATS ET LES CHIENS

(30) Priority: 31.08.2010 US 378457 P
(43) Date of publication of application: 10.07.2013
(73) Proprietor: Gallant Pet, Inc., Santa Monica, CA 90401 (US)
(72) Inventor: WOODS, Erik, John, Indianapolis, IN 46202 (US); THIRUMALA, Sreedhar, Indianapolis, IN 46250 (US); ZACHARIAS, Shelly, J., Indianapolis, IN 46236 (US)
(74) Representative: Mathys & Squire
(86) International application number: PCT/US2011/049977
(87) International publication number: WO 2012/030968

(56) References cited:
- WO-A1-2005/045008
- US-A1- 2002 064 519
- US-A1- 2008 175 821
- US-A1- 2008 241 112
- US-A1- 2009 214 484
- US-A1- 2009 214 485
- US-A1- 2010 098 672

## Description

### FIELD OF INVENTION

The present disclosure relates to therapies using allogeneic, mesenchymal stem cells for the treatment of diseases in animals, with particular emphasis on canine, feline, equine, human, and lagomorph species.

### BACKGROUND

Mesenchymal stem cells, multipotent mesenchymal stromal cells, and/or mesenchymal-like stem cells (all referred to herein as "MSCs") derived from human origin are known to possess the potential for multiple differentiation abilities *in vitro* and *in vivo,* although the source of those cells, e.g., the tissue and species from which those cells are harvested, have proven to significantly affect the pluripotent capability of those cells. Thus, while MSCs offer great therapeutic promise for a diverse range of medical applications, the range of differentiation, and therefore, therapeutic utility, appears to be related to the tissue and species origin of the MSC.

For instance, in humans, MSCs have been shown to express a broad spectrum of differentiation potential from cell types of mesodermal origin, like osteoblasts, adipocytes, chondrocytes to ectodermal (neuronal) and endodermal (hepatocytes) origins in response to chemical, hormonal or structural stimuli. However, to date, the most common source of MSC for therapeutic treatment is bone marrow, despite the fact that isolation of MSCs from bone marrow results in: smaller number of available MSCs as compared to other sources, severe discomfort to the patient during isolation, and a drastic decrease in the available numbers of MSCs with age. Further, while some insight into the use of stem cells for therapeutic treatment has been gained in recent years, there is still a great deal to be learned about the efficacy and dosage of particular MSCs in treating specific diseases and diseased states.

Further, in order for a therapeutic treatment utilizing MSCs to be practical, there must be an available supply of the MSCs necessary for the treatment at the time the individual requires the therapy. As noted above, MSCs originating from certain tissues may be in short supply from the donor, or may result in severe discomfort or negative side effects to the donor.

As such, it would be greatly appreciated to identify sources of MSCs that are readily available for harvest during routine procedures, and which may effective in treating specific diseases or disease states in a given species, and particularly in humans and companion animals. US 2009/214484 describes stem cell therapy for the treatment of central nervous system disorders.

US 2009/214485 describes stem call therapy for the treatment of diabetic retinopathy and diabetic optic neuropathy.

US 2008/0175821 relates to a neuronal regeneration material screening method using ex-vivo models, more precisely a neuronal regeneration material screening method using an organotypic spinal cord slice culture. WO 2005/045008 describes a method for inducing chondrogenesis on mesenchymal stem cells.

EP2105498 relates to a therapeutic composition for atopic dermatitis.

WO 2008/020815 discloses a method of preparing a conditioned cell culture medium.

WO 2006/121445 discloses therapy of kidney diseases and multi-organ failure with mesenchymal stem cells and mesenchymal stem cell conditioned media.

US 2002/064519 describes methods and devices using non-autologous mesenchymal stem cells.

### SUMMARY

Based on the disclosure contained herein, the present invention provides a mesenchymal stem cell composition for use in treating a canine or feline patient suffering from degenerative bone disease, osteoarthritis, rheumatoid arthritis, polyarthritis, systemic lupus erythematosus, inflammatory bowel disease, atopy, hepatitis, chronic steroid responsive meningitis-arteritis, beagle pain syndrome, chronic renal failure disease, keratoconjunctivitis sicca, immune mediated non-erosive arthritis, immune mediated hemolytic anemia, immune mediated thrombocytopenia, Evans syndrome, intervertebral disc disease, refractory corneal ulcer, diabetes mellitus, eosinophilic granuloma complex, cholangitis, exercise induced pulmonary hemorrhage, rhabdomyolysis, corneal ulcer, eczema, multiple sclerosis, muscular dystrophy, and muscle fibrosis secondary to disease or trauma, wherein the treatment comprises the step of systemically administering a therapeutic dose of a mesenchymal stem cell composition through an intravenous injection, the mesenchymal stem cell composition comprising mesenchymal stem cells harvested from at least one tissue selected from the group consisting of testicle tissue and uterine tissue, wherein the mesenchymal stem cell composition comprises a solution having a concentration of approximately 2,000,000 cells or less per mL of solution, wherein the mesenchymal stem cells are allogeneic or autologous to the patient.

The present invention further provides a mesenchymal stem cell composition for use in treating a canine or feline patient suffering from a preselected disease or diseased state, wherein the treatment consists of the step of systemically administering a therapeutic dose of a mesenchymal stem cell composition through an intravenous injection, the mesenchymal stem cell composition comprising mesenchymal stem cells harvested from at least one tissue selected from the group consisting of testicle tissue and uterine tissue, wherein the mesenchymal stem cell composition comprises a solution having a concentration of approximately 2,000,000 cells or less per mL of solution, and wherein the mesenchymal stem cells are allogeneic or autologous to the patient, and wherein the preselected disease or diseased state is selected from the group consisting of degenerative bone disease, osteoarthritis, rheumatoid arthritis, polyarthritis, systemic lupus erythematosus, inflammatory bowel disease, atopy, hepatitis, chronic steroid responsive meningitis-arteritis, beagle pain syndrome, degenerative myelopathy, chronic renal failure disease, keratoconjunctivitis sicca, immune mediated non-erosive arthritis, immune mediated hemolytic anemia, immune mediated thrombocytopenia, Evans syndrome, intervertebral disc disease, refractory corneal ulcer, diabetes mellitus, spinal trauma, eosinophilic granuloma complex, cholangitis, exercise induced pulmonary hemorrhage, rhabdomyolysis, corneal ulcer, eczema, multiple sclerosis, muscular dystrophy, spinal injury, and muscle fibrosis secondary to disease or trauma.

The present invention and embodiments thereof are set out in the appended claims.

Additional embodiments, objects, and features of the invention will be apparent from the description which follows.

### DETAILED DESCRIPTION

According to one instance, a method for treating a specified disease or diseased state comprises the intravenous (IV) injection of a therapeutic dose of preselected MSCs to a patient suffering from an identified disease or diseased state. Systemic treatment through IV injection has proven to reduce invasiveness, improve the speed and cost of the procedure, and decrease morbidity and recovery time when compared to site-specific application.

It will be appreciated that a method for treating a specified disease or diseased state, according to certain instances, comprises one or more doses of the preselected MSCs at preselected time intervals. By way of nonlimiting example the preselected MSCs are delivered at approximately weekly intervals, at approximately two week intervals, at approximately three week intervals, at approximately monthly intervals, at approximately two month intervals, at approximately three month intervals, at approximately four month intervals, at approximately five month intervals, or at approximately six month intervals.

In at least one instance, the therapeutic dose of each IV injection of preselected MSCs comprises about six million MSCs per kg of the patient's body weight up to a maximum of fifty million MSCs regardless of body weight. In order to obtain the necessary number of MSCs, preselected MSCs are collected and expanded utilizing cell culture techniques described in further detail below, and those expanded MSCs are harvested and segregated into cell counts of approximately six million to about fifty million cells, as needed. As used herein, a therapeutic dose means the number of MSCs of sufficient quantity to decrease the physiological symptoms of the specified disease or diseased state in the patient.

Thereafter, according to one instance, those segregated cells are diluted into a balanced saline solution or other suitable dilutive solution such that each diluted population of cells has a concentration of approximately 2,000,000 cells or less per mL of solution. According to yet another instance, | each cell count is diluted to a concentration of approximately 1,000,000 cells or less per mL of solution; approximately 500,000 cells or less per mL of solution; approximately 250,000 cells or less per mL of solution; and approximately 100,000 cells or less per mL of solution. It will be appreciated that the diluted population of cells may be harvested such that they are free of substantially all of the culture medium upon which the MSCs were expanded, or the cells may be harvested to include at least a portion of the cell conditioned medium upon which the cells were expanded. Likewise, the diluted population of cells may comprise additional physiological electrolyte additives. Alternatively, the medium may also be delivered free of cells.

In certain instances, preselected MSCs are autologous or allogeneic to the patient, and those preselected MSCs may be isolated from a donor during health procedures that are unrelated to the purpose of harvesting MSCs. As such, MSCs may be harvested in a manner that does not adversely affect the donor or result in unnecessary medical treatments to the donor. For instance, placental, uterine, umbilical cord, and testicle MSCs may be harvested during routine birthing procedures or during spay/neuter treatments for dogs and cats, with those tissues being banked for later expansion or later use. Further, it will be appreciated that donors are preferably screened to ensure that the donor is in good general health, and may be screened for the presence of diseases, current status of vaccinations, or presence of antibiotics in the donor's system as necessary.

Preselected MSCs include dental derived stem cells such as stem cells harvested from dental pulp, periodontal ligaments, and other dental tissues; stem cells harvested from testicle tissue; stem cells harvested from bone marrow; stem cells harvested from placental tissue; stem cells harvested from uterine tissue (including endometrial regenerative cells), stem cells harvested from umbilical cord tissue, and stem cells harvested from full thickness skin biopsies.

An identified disease or diseased state includes degenerative bone disease, osteoarthritis, rheumatoid arthritis, polyarthritis, systemic lupus erythematosus, inflammatory bowel disease, atopy, hepatitis, chronic steroid responsive meningitis-arteritis, beagle pain syndrome, degenerative myelopathy, chronic renal failure disease, dilated and mitral cardiomyopathy, keratoconjunctivitis sicca, immune mediated non-erosive arthritis, immune mediated hemolytic anemia, immune mediated thrombocytopenia, Evans syndrome, intervertebral disc disease, muscle fibrosis secondary to disease or trauma, refractory corneal ulcer, diabetes mellitus, spinal trauma, eosinophilic granuloma complex, hypertrophic cardiomyopathy, cholangitis, spinal injury, exercise induced pulmonary hemorrhage, rhabdomyolysis, corneal ulcer, eczema, multiple sclerosis, muscular dystrophy, spinal injury, diabetes mellitus, hepatitis, myocardial infarction, congestive heart failure, and muscle fibrosis secondary to disease or trauma.

Further, according to at least one embodiment, a method for treating a specified disease or diseased state comprises the topical administration of a therapeutic dose of preselected MSCs or MSC conditioned media to a patient suffering from an identified disease or diseased state and/or in need of treatment for the identified disease or diseased state. According to at least one example, a suspension of MSCs in a saline solution is applied topically to a patient suffering from atopy or eczema. According to certain instances, the suspension of MSCs further contains portions of the cell conditioned media upon which the MSCs were cultured, and may contain an agent for making the suspension thicker, such as a colloid or hydrogel. According to certain instances, a method for treating a specified disease comprises the topical administration of a suspension of cell conditioned media (sometimes referred to as "spent media") without the MSCs.

### EXAMPLES

### A. Methods for Extracting and Processing MSCs from Selected Tissue.

The present application contemplates the collection and delivery of a naturally occurring population of cells derived from placental/umbilical cord, bone marrow, skin, or tooth pulp tissue. The designated name for this population is mesenchymal stem cells, or "MSCs". MSCs are a population of adherent multipotent mesenchymal stromal cells originating from the mesoderm with some populations (such as those from teeth) having potential ectoderm origin as well (e.g. ecto-mesenchymal). The MSCs are generally an adherent cell population expressing markers CD90 and CD105 (>90%) and lacking expression of CD34 and CD45 and MHC class II (<5%) as detected by flow cytometry. It will be appreciated that each of the MSCs were extracted and processed from the preselected tissues as noted below.

### 1. Placental, Testicle, and Uterine Tissue.

Placenta was collected from delivery procedures, and testicle and uterine tissue was collected from spay or neuter procedures. Regardless of which tissue was collected, the tissue was placed in sterile containers with phosphate buffered saline ("PBS"), penicillin/streptomycin and amphotericin B. Specifically, harvested tissue was first surface sterilized by multiple washes with sterile PBS, followed by immersion in 1% povidoneiodine ("PVP-1") for 2 minutes, immersion in 0.1% sodium thiosulfate in PBS for 1 minute, and another wash in sterile PBS. Next the tissue is dissected into 5g pieces for digestion. Enzymatic digestion was performed using a mixture of collagenase type I and type II along with thermolysin as a neutral protease. The digestion occurred in a 50cc sterile chamber for 20-45 minutes until the tissue was disaggregated and the suspending solution was turbid with cells. Next the solution was extracted leaving behind the matrix, and cold (4°C) balanced salt solution with fetal bovine serum ("FBS") at 5% concentration was added to quench the enzymes. This resulting suspension was centrifuged at 600 × g, supernatant is aspirated and MESENCULT^{®} complete medium (basal medium containing MSC stimulatory supplements available from StemCell Technologies, Vancouver, British Columbia) was added to a final volume of approximately 1.5 times the digestion volume to neutralize the digestion enzymes. This mixture was centrifuged at 500 g for 5 minutes, and the supernatant aspirated. The cell pellet was be re-suspended in fresh MESENCULT^{®} complete medium plus 0.25 mg/mL amphotericin B, 100 lU/mL penicillin-G, and 100 mg/mL streptomycin (JR Scientific, Woodland, CA). Cells were plated at an initial concentration of one starting 5g tissue digest per 225 cm2 flask. Culture flasks were monitored daily and any contaminated flasks removed immediately and recorded. Non-contaminated flasks were monitored for cell growth, with medium changes taking place three times per week. After 14 days of growth, MSC were detached using 0.25% trypsin/lmM EDTA (available from Invitrogen, Carlsbad, CA). Cell counts and viability were assessed using flow cytometry techniques and cells were banked by controlled rate freezing in sealed vials.

### 2. Bone Marrow.

Bone marrow was collected and placed within a "washing tube". Before the collection procedure a "washing tube" is prepared in the class 100 Biological Safety Cabinet in a Class 10,000 GMP Clean Room. To prepare the washing tube, 0.2 mL amphotericin B (Sigma-Aldrich, St Louis, MO), 0.2 mL penicillin/streptomycin (Sigma 50 ug/nl) and 0.1 mL EDTA-Na2 (Sigma) were added to a 50 mL conical tube (Nunc) containing 40 mL of GMP-grade phosphate buffered saline (PBS). Specifically, the washing tube containing the collected bone marrow was topped up to 50 mL with PBS in a class 100 Biological Safety Cabinet and cells was washed by centrifugation at 500 g for 10 minutes at room temperature, which produced a cell pellet at the bottom of the conical tube. Under sterile conditions supernatant was decanted and the cell pellet was gently dissociated by tapping until the pellet appeared liquid. The pellet was re-suspended in 25 mL of PBS and gently mixed so as to produce a uniform mixture of cells in PBS. In order to purify mononuclear cells, 15 mL of Ficoll-Paque (Fisher Scientific, Portsmouth NH) density gradient was added underneath the cell-PBS mixture using a 15 mL pipette. The mixture was subsequently centrifuged for 20 minutes at 900 g. Thereafter, the buffy coat was collected and placed into another 50 mL conical tube together with 40 mL of PBS. Cells were then centrifuged at 400g for 10 minutes, after which the supernatant was decanted and the cell pellet re-suspended in 40 mL of PBS and centrifuged again for 10 minutes at 400g. The cell pellet was subsequently re-suspended in 5 mL complete DMEM-low glucose media (GibcoBRL, Grand Island, NY) supplemented with 20% Fetal Bovine Serum specified to have Endotoxin level less than or equal to 100 EU/mL (with levels routinely less than or equal to 10 EU/mL) and hemoglobin level less than or equal to 30 mg/dl (levels routinely less than or equal to 25 mg/dl). The serum lot used was sequestered and one lot is used for all experiments. Additionally, the media was supplemented with 1% penicillin/streptomycin, 1% amphotericin B, and 1% glutamine. The re-suspended cells were mononuclear cells substantially free of erythrocytes and polymorphonuclear leukocytes as assessed by visual morphology microscopically. Viability of the cells was assessed with trypan blue. Only samples with >90% viability were selected for cryopreservation in sealed vials.

### 3. Tooth Pulp.

Teeth were extracted under sterile conditions and placed into sterile chilled vials containing 20mL of phosphate buffered saline with penicillin/streptomycin and amphotericin B (Sigma-Aldrich, St. Louis, MO). Teeth were thereafter externally sterilized and processed first by washing several times in sterile PBS, followed by immersion in 1% povidoneiodine (PVP-1) for 2 minutes, immersion in 0.1% sodium thiosulfate in PBS for 1 minute, followed by another wash in sterile PBS. The roots of cleaned teeth were separated from the crown using pliers and forceps to reveal the dental pulp, and the pulp was placed into an enzymatic bath consisting of type I and type II collagenase (Vitacyte, Indianapolis, USA) with thermolysin as the neutral protease. Pulp tissue was allowed to incubate at 37°C for 20-40 min to digest the tissue and liberate the cells. Once digestion was complete, MESENCULT^{®} complete medium was added to a final volume of 1.5× the digestion volume to neutralize the digestion enzymes. This mixture was centrifuged at 500 g for 5 min, and the supernatant aspirated. The cell pellet were re-suspended in fresh MESENCULT^{®} complete medium plus 0.25 mg/mL amphotericin B, 100 IU/mL penicillin-G, and 100 mg/mL streptomycin (JR Scientific, Woodland, CA). Cells were plated at an initial concentration of one tooth digest per 25 cm² flask. Culture flasks were monitored daily and any contaminated flasks removed immediately and recorded. Non-contaminated flasks were monitored for cell growth, with medium changes taking place three times per week. After 14 days of growth, MSC were detached using 0.25% trypsin/1mM EDTA (Invitrogen, Carlsbad, CA), cell counts and viability were assessed using a standard trypan blue dye exclusion assay (Sigma) and hemacytometer, and bAU3 the DPSC divided equally between two 75 cm² flasks. After the first passage, DPSC cultures were harvested once they reach 70-80% confluence. These cells were cryopreserved in sealed vials.

### 4. Skin Tissue.

MSCs from the skin, including epidermal, dermal, and subcutaneous tissue of healthy adult patients undergoing cosmetic plastic surgery were isolated by collagenase digestion procedure. Once received, the tissue was cleaned of any unwanted adipose tissue and hair. The tissue was then sterilized using 1X PVP-iodine solution and 1X sodium thiosulfate followed by washing twice in sterile PBS. The dermis was then minced into 1 mm³ pieces following collagenase enzymatic digestion for 30-40 minutes at 37°C. Afterwards, tissue pieces were dissociated by pipetting into 5 mL pipette and centrifuged at 300g for 5 min. The pellet was suspended in cell growth media Dulbecco's Modified Eagle Medium: Nutrient Mixture F-12 ("DMEM/F12") (available from Invitrogen, Carlsbad, CA) (1:1) containing amphoterecin, penicillin and streptomycin supplemented with 10% fetal bovine serum. Cell suspensions were transferred into T-25 tissue culture flask and grown until 80-90% confluence. The cells were placed in a T-75 flask before being used for flow analysis and differentiation.

### 5. Umbilical Cord Tissue.

MSCs from the umbilical cord were harvested during delivery. Once received, the tissue was washed two to three times in sterile PBS and then divided into pieces of approximately 5 grams each. Thereafter, the tissue was decontaminated, and each 5 gram aliquot of tissue was placed in a sterile 100 mm tissue culture dish, and covered with a lid to prevent drying. The tissue was dissociated via enzymatic digestion in 50 cc tubes, and was minced into fragments less than 1 mm³ using a sterile scalpel. Then, the chopped tissue was placed in an enzyme bath, and the tube was capped and transferred to an incubator. The tubes were swirled for fifteen seconds every ten minutes for forty minutes. Thereafter, the digesting enzyme was diluted by adding 45 mL of cold DME/F12 complete media (FBS, PenJStrep and Amphotericin B), with the tubes being capped and inverted to mix the contents. Next, the tubes were centrifuged at 400 × g for fifteen minutes on low break. The top media was aspirated using a 25 mL pipette by leaving approximately 5 mL at the bottom of the tube, with special care being taken to aspirate the entire medium in the tube. The bottom 5 mL medium (contaiing tissue fragments and cells including MSCs) was resuspended in fresh 20 mL DME-F12 complete medium mixed well and placed into a t-75 flask, and transferred to an incubator. The tissue was washed off during the first media change after 48 hours post-digestion, and the media was changed three times per week. Cells were grown to 70%-80% confluence and then either passaged, frozen down as passage zero cells, or differentiated. Cells were not allowed to reach confluence or to remain at confluence for extended periods of time.

### B. Methods for Expanding Cell Populations.

Cell expansion for cells originating from any of the abovementioned tissues above took place in clean room facilities purpose built for cell therapy manufacture and meeting GMP clean room classification. In a sterile class II biologic safety cabinet located in a class 10,000 clean production suite, cells were thawed under controlled conditions and washed in a 15 mL conical tube with 10 ML of complete DMEM-low glucose media (cDMEM) (GibcoBRL, Grand Island, NY) supplemented with 20% Fetal Bovine Serum (Atlas) from dairy cattle confirmed to have no BSE % Fetal Bovine Serum specified to have Endotoxin level less than or equal to 100 EU/mL ( with levels routinely less than or equal to 10 EU/mL) and hemoglobin level less than or equal to 30 mg/dl (levels routinely less than or equal to 25 mg/dl). The serum lot used was sequestered and one lot was used for all experiments.

Cells were subsequently placed in a T-225 flask containing 45 mL of cDMEM and cultured for 24 hours at 37°C at 5% CO2 in a fully humidified atmosphere. This allowed the MSC to adhere. Non-adherent cells were washed off using cDMEM by gentle rinsing of the flask. Adherent cells were subsequently detached by washing the cells with PBS and addition of 0.05% trypsin containing EDTA (Gibco, Grand Island, NY, USA) for 2 minutes at 37°C at 5% CO2 in a fully humidified atmosphere. Cells were centrifuged, washed and plated in T-225 flask in 45 mL of cDMEM.

This resulted in approximately 6 million cells per initiating T-225 flask. The cells of the first flask were then split into 4 flasks. Cells were grown for 4 days after which approximately 6 million cells per flask were present (24 million cells total). This scheme was repeated but cells were not expanded beyond 10 passages, and were then banked in 6 million cell aliquots in sealed vials for delivery.

All processes in the generation, expansion, and product production were performed under conditions and testing that was compliant with current Good Manufacturing Processes and appropriate controls, as well as Guidances issued by the FDA in 1998 Guidance for Industry: Guidance for Human Somatic Cell Therapy and Gene Therapy; the 2008 Guidance for FDA Reviewers and Sponsors Content and Review of Chemistry, Manufacturing, and Control (CMC) Information for Human Somatic Cell Therapy Investigational New Drug Applications (INDs); and the 1993 FDA points-to-consider document for master cell banks were all followed for the generation of the cell products described.

Donor cells were collected in sterile conditions, shipped to a contract manufacturing facility, assessed for lack of contamination and expanded. The expanded cells were stored in cryo vials of approximately 6 million cells/vial, with approximately 100 vials per donor. At each step of the expansion quality control procedures were in place to ensure lack of contamination or abnormal cell growth.

In another aspect, cells are grown in media and the cells, along with the media, are recovered after about 5-10 days. The cells are prepared in this "conditioned" media for transfusion at concentrations of less than about 100,000 cells per mL. Physiological electrolyte additives may be added. The cell solution is administered intravenously.

In a further method, cells are grown in media for about 5-10 days. This media is then transfused intravenously without cells or given locally to the site of the injury. Further methods involve isolation and/or concentration of stem cell produced factors and/or further refinements of these chemicals and/or compounds.

### C. Description of Diseases and Diseased States.

MSC's can be administered to an human/animal in need of treatment for one, or one or more, of the following diseases or diseased states, or others mentioned herein, potentially with or without need for treatment for any other diseases or disease states that could be treated with the MSC's.

### 1. Arthritis

Arthritis is classified as non-inflammatory or inflammatory based on joint fluid analysis. Degenerative joint disease/osteoarthritis ("DJD/OA") is considered noninflammatory, and displays degenerative changes in the joint with lack of fever, leukocytosis or other systemic signs. Osteoarthritis ("OA") is a breakdown of articular cartilage causing increased edema in the joint, osteophyte formation and fibrosis of the periarticular soft tissues. The end result is loss of elasticity, joint degeneration and instability. A series of changes in the articular cartilage that cause inflammation can eventually lead to complete loss of cartilage. The remodeling and inflammatory changes create pain that decrease the mobility of the affected joint therefore muscle atrophy results.

Osteoarthritis is further classified as primary or secondary depending on the etiology. Primary OA is due to cartilage degeneration in aging pets and occurs for unknown reasons. Secondary OA, which is more common than primary, occurs in response to an injury, abnormality or disease that causes joint instability. Either form of the disease is always considered progressive regardless of cause or treatment. Arthritis and OA are found in all breeds and ages of dogs and cats.

### 2. Rheumatoid Arthritis.

Rheumatoid arthritis ("RA") is considered an inflammatory joint disease due to inflammatory changes that occur in the synovium along with systemic clinical signs. Inflammatory joint disease is further classified as infectious or immune mediated, and immune mediated disease is considered erosive or non-erosive. RA is an inflammatory, noninfectious, erosive, immune mediated polyarthritis of dogs where the synovial membrane proliferates. While the pathogenesis is not fully understood, it is characterized by the prostaglandins causing erosion of the subchondral bone beginning at the joint margins in turn causing granulation tissue to invade the bone. Antibodies called rheumatoid factors (IgG, IgM and IgA) are produced against an antigen (IgG) which produce joint inflammation. Thereafter, the synovial membrane thickens, fibrosis occurs, pannus (vascular tissue) invades the joint and releases proteolytic enzymes causing erosion of articular cartilage and subchondral bone. The articular surface then collapses which destabilizes the joint leading to subluxation or luxation that appears as a deformed joint on physical exam and radiographs.

Clinical signs vary depending on the stage of the disease. In early stages the patient shows shifting leg lameness, possible low grade fever, inappetance, and mild lymphadenopathy. Lameness later becomes more severe along with the other clinical signs. Radiographic changes usually are not visible for first few weeks until detailed radiographs show cyst like lucent lesions in the subchondral bone. Later in the disease degenerative radiographic changes are obvious as are worsening clinical signs of joint swelling subcutaneous nodules, and significant joint pain.

Along with clinical signs and radiographic findings, rheumatoid factor ("RF") may be found in serum or joint fluid although up to 30% of dogs with RA will test seronegative. Therefore a negative RF does not rule out the disease and a positive RF does not definitive diagnose RA due to false positives in non RA animals with other inflammatory diseases elsewhere in the body. The age of onset of RA is 1-9 years with an average of 4-5 years. Small breed dogs are most commonly affected, with poodles and shelties over represented.

### 3. Degenerative Radiculomyelopathy.

Degenerative radiculomyelopathy and German Shepherd degenerative myelopathy are slowly progressive neurologic disorders of unknown etiology affecting most commonly middle age to older large breed dogs. A gradual loss of white matter of the spinal cord and myelin causing ataxia/weakness and eventually paraparesis of the pelvic limbs is observed. This disease most often occurs in middle-age to older large breed dogs but has been found all breeds/mixed breeds, no sex predilection, in some young animals and in cats. German Shepherd breeds appear to be over represented.

Early in the disease the pet has difficulty getting up from a lying or sitting position. The back end will sway or the gait will appear ataxic. The pelvic limbs may begin to criss-cross over one another. As the disease progresses, the pet will drag her toenails on the ground when walking. One characteristic of affected patients includes nails wearing down from dragging the hind feet. Generalized weakness of the hind end of the animal becomes more obvious, more difficulty rising and more difficulty getting stable on slick flooring. However, the disease does not generally result in pain to the patient.

### 4. Chronic Renal Failure.

Chronic renal failure ("CRF") is defined as a progressive, irreversible renal dysfunction that occurs over months to years. Chronic disease is differentiated from acute based on patient history, physical exam and/or laboratory findings to suggest the disease has been present for an extended period. CRF is characterized by azotemia along with a low urine specific gravity (dogs<1.030, cats<1.035) for a prolonged period of time. These animals also have a 75% reduction in their functional renal mass. CRT will continue to progress negatively even after any possible inciting cause is removed. Clinical signs include poor appetite, poor hair coat, polyuria, polydipsia, weight loss, usually small kidney size on palpation and radiographs unless polycystic disease or neoplastic disease, osteodystophy may be present most often in the jaw (rubber jaw), pale mucus membranes from non-regenerative anemia, oral ulcers, acute blindness, cervical ventroflexion, hypothermic, hypertensive.

### 5. Dilated Cardiomyopathy.

Dilated cardiomyopathy ("DCM") is characterized by diminished contractile dysfunction and cardiac chamber dilation. It is the second most common form of heart disease in the dog. DCM is idiopathic, and possibly genetic due to it being found as an inherited autosomal recessive trait in the Portuguese water dog who is the only canine breed to show a juvenile form of DCM. Other canine breeds that are overrepresented include the Boxer, Doberman Pinscher, Great Dane, Newfoundland and Irish Wolfhound. These breeds are middle age when clinical signs, usually congestive heart failure, occur. Contractile dysfunction leads to congestive heart failure ("CHF"). CHF is not purely a consequence of impaired pump function, but is also a neuroendocrine syndrome in which activation of the adrenergic nervous system and specific endocrine pathways such as the rennin-angiotensin-aldosterone system play an integral role. When cardiac function declines, compensatory mechanisms activate to maintain systemic perfusion, pressures and cardiac output.

### 6. Chronic Hepatitis,

Chronic hepatitis ("CH") morphology is characterized by hepatocellular apoptosis or necrosis, mixed inflammatory cells and/or variable mononuclear cells and fibrosis. Many causes of hepatitis exist but the underlying cause of chronic hepatitis is often undetermined. Causes of hepatitis can include copper storage disease which allows an animal to accumulate abnormal levels of copper in the liver until toxicity occurs. It is an inherited disease found in, amongst others, mixed breed and pure breed dogs including Bedlington Terriers, West Highland White terriers, Doberman Pinchers, Skye Terriers, Dalmatians and Labrador Retrievers. Infectious diseases have been associated with hepatitis such as leptospirosis and canine viral hepatitis. CH usually occurs between 4-10 years of age, with chronic hepatitis being more common in female dogs.

### 7. Atopy, Eczema.

Atopy (inhalant dermatitis) is a hypersensitivity reaction to inhaled or cutaneously absorbed environmental antigens in individuals who are genetically predisposed. Age of onset can be from 6 months to 6 years with the average being between 1-3 years. Clinical signs include skin erythema, generalized or local pruitis that can be seasonal or non-seasonal, areas of moist dermatitis, papules, pustules, ulcerative eruptions, scales, hyperpigmentation, lichcnification or alopecia. Self-trauma can result in secondary skin lesions including excoriations, saliva staining, lick granuloma or even open wounds. Atopy can also manifest as chronic otitis externa, conjunctivitis, epiphora, allergic bronchitis, rhinitis, secondary pyoderma, Malassezia dermatitis, chronic acral lick dermatitis or rarely hyperhidrosis.

Similarly, eczema is a general term for any type of dermatitis or inflammation of the skin. Eczema will cause pruritus (itching) and redness of the skin with blistering, weeping and/or peeling being possible. There are several skin diseases that are considered eczemas with Atopic Dermatitis (AD) or Atopy being the most severe and chronic disease on the list. AD is present worldwide but seems more common in developed countries affecting men and women equally of all races. AD is not contagious but is inherited; the clinical signs often develop during infancy or early childhood the majority of the time. Some children are fortunate enough to "outgrow" eczema but most are affected for a lifetime. The clinical signs of AD are dry, scaly, itchy skin, cracks in the skin, and rashes on the cheeks, arms and legs. The symptoms are episodic, during a flare up it is not uncommon to develop open weeping or crusted sores from infection or self-excoriation. Eczema usually affects the insides of the elbows, the back of the knees and the face but can cover most of the body. People who have atopic dermatitis often either have family members or they themselves suffer from asthma, hay fever or both; these three diseases together are referred to as the "Atopy triad."

There are trigger factors that people are exposed to which worsen or cause their AD to flare-up. Trigger factors are substances or conditions such as dry skin, allergens including food or environmental, stress, extreme climate changes, exercise (heat/sweat) and numerous irritants such as smoke, fumes, fragrances, detergents, etc. When people with AD are exposed to a trigger factor which they are sensitive to, an over production of inflammatory cells migrate to the skin causing a pruritic and painful reaction, which in turns causes the person to scratch worsening the reaction. The underlying pathophysiology of AD is an over response of the immune system to an allergen or irritant.

Traditional treatment of Eczema or AD has been topical creams that modify the skin's immune response and thereby inflammatory response (hydrocortisone can be used only short term, tacrolimus or pimecrolimus), skin moisturizers (petroleum based), behavior modification to avoid irritants and in more advanced cases short term injections or oral use of steroids are used. People with AD are prone to skin infections, namely staph and herpes, therefore they are taught to watch for clinical signs of skin infection and if suspected, consult with their doctor immediately to avoid aggravating the disease. Recent studies have identified direct links between canine atopy and the human form of atopic dermatitis making the naturally occurring disease in the canine an ideal model for analogous human studies.

### 8. Keratoconjunctivitis Sicca.

Keratoconjunctivitis sicca ("KCS") is a common ocular disease in the dog characterized by decreased aqueous tear production that can result in corneal and conjunctival pain and disease. KCS can affect the vision, especially if left untreated, blindness or loss of the globe can occur. KCS has many underlying etiologies but the most common cause is immune mediated lacrimoadenitis. Based on a positive response to immunomodulation therapy, greater than 75% of canine KCS cases are due to this immune mediated inflammation of the lacrimal gland. This immune mediated etiology also appears to be highly breed related and is most common in those dogs with atopic skin disease such as the Golden Retriever. Other causes of KCS include: a) congenital lacrimal gland atresia that do not respond to immune modulating drugs and breeds commonly affected include the Yorkies, Beagles, Miniature Pinschers and Miniature Dachshunds; b) neurogenic KCS can be seen in animals that have had severe otitis externa/media/interna that now have lack of parasympathetic innervation to the lacrimal and third eyelids; c) ocular surface infections (primary or secondary to systemic disease, distemper; d) iatrogenic KCS from amputation of third eyelid for removal of cherry eye; and f) certain drug therapy can cause decreased tear production. Breeds that are over represented include English Bulldogs, American Cocker Spaniels, West Highland White Terriers, Lhasa Apsos, Shih Tsus, Pugs, Pekingese, Boston Terriers, Cavalier King Charles Spaniels, Yorkshire Terriers and Miniature Poodles.

### 9. Systemic Lupus Erythematosus.

Systemic Lupus erythematosus (SLE) is a multi-systemic immune-mediated disease in which antibodies are directed against the body's tissues and circulating immune complexes are deposited affecting multiple systems. The most common affected areas are the joints, kidneys and skin. SLE is the result of a Type III hypersensitivity reaction although it may also be associated with type II and IV reactions. SLE is an immune complex deposition disease and can also cause heightened antibody responsiveness with a tendency to produce autoantibodies. Usually either the immune complex or the autoantibody aspect of the disease predominates in the animal.

Clinical signs can be severe and variable but considering the most common systems affected, the most common clinical signs of the immune complex SLE are: lameness, fever, pain (polyarthritis), polyuria/polydipsia, anorexia, nausea dehydration (renal disease) and dermatological (mucocutaneous) disorders. The dermatological signs are one of the most common and extremely diverse clinical signs in animals with SLE. Skin lesions tend to be symmetrical and most commonly affect the mucocutaneous junctions of the body, the feet and the ears. The skin lesions will often present as erythematous, crusty dermatitis. The most common disorders associated with the autoimmune aspect of SLE are hemolytic anemia and thrombocytopenia. With either variety of SLE multiple organ systems may become involved, including the cardiovascular system and central nervous system. Psychosis has even been reported in animals with SLE similarly to human patients with SLE. Females are at slightly greater risk than males. German Shepherds, Collies and Shetland Sheepdogs are thought to be at greater risk. More than 40% of dogs diagnosed with SLE succumb to the disease within one year of diagnosis. SLE is rare but has been documented in cats and large animals.

### 10. Immune-mediated Thrombocytopenia and Immune-mediated Hemolytic Anemia.

Immune-mediated thrombocytopenia ("IMT") and Immune-mediated hemolytic anemia ("IMHA") can occur in the cat and dog as primary or secondary disease. When both arise simultaneously the disorder is referred to as Evans syndrome. IMT is a common cause of non-traumatic bleeding in small animals and IMHA is a common cause of anemia. Primary IMT and IMHA is the result of an autoimmune disorder, while secondary IMT or IMHA can result in response to a variety of infectious, inflammatory or neoplastic diseases or can be attributed to drug insult. IMT is most commonly secondary to infectious or neoplastic disease in cats, unlike IMT in dogs. The mechanism of destruction of red blood cells in IMHA is antibody mediated cytotoxic (Type II). IMHA affects young to middle aged animals with Cocker Spaniels, English Springer Spaniels, Poodles and Old English Sheepdogs being overrepresented in the dog population. IMT is most often seen in middle aged female dogs with average age of onset being 6 years.

IMHA clinical signs can range from non-clinical signs of anemia such as pale mucus membranes to more significant disease including lethargy, weakness, a hemic heart murmur, often compensatory tachycardia, tachypnea and bounding pulses are noted. Some patients will have ongoing immunological or inflammatory disease clinical findings such as fever or anorexia or less commonly lymphadenopathy. Jaundice (icterus) is a common finding in IMHA patients due to extravascular hemolysis. Pulmonary thromboembolism is a common complication of IMHA with severe anemia, especially if on aggressive steroid treatment. IMT appears as spontaneous hemorrhage in otherwise healthy appearing dogs. Cats may have other clinical symptoms of a primary disorder. Questioning of the owner may uncover previous minor episodes of bleeding. The hallmark lesion of IMT in any species is petechial hemorrhage that may merge into ecchymosis.

### 11. Steroid Responsive Meningitis-arteritis.

Steroid responsive meningitis-arteritis ("SRMA") is of unknown etiology but is thought to be immune mediated in origin. SRMA is also known as juvenile polyarteritis, necrotizing vasculitis, canine juvenile polyarteritis syndrome, and Beagle Pain Syndrome. SRMA arises in juveniles, with no sex predilection, and most notably found in the Beagle. SRMA may also be found in other breeds including Bernese Mountain dogs, German Shorthaired Pointers, Boxers, Toller Retrievers and mixed breeds. Two forms of the disease are described: acute/fulminating and chronic. The acute form is characterized by neutrophilic pleocytosis of the cerebrospinal fluid and the chronic form by mononuclear or mixed cell pleocytosis accompanied with neurological deficits. Either form may have systemic necrotizing vasculitis with severe subarachnoid hemorrhages throughout the length of the spinal cord and brain stem. Thrombosis and vascular occlusion may lead to neural ischemia. Affected vessels may contain cells with IgG and hemosiderin filled macrophages. Amyloidosis and systemic vasculitis may occur in some dogs. SRMA is known to affect medium to large breed dogs usually less than 2 years of age but as old as 7 years. With early aggressive immunosuppressive treatment therapy approximately 60-80% of dogs are cured with 20-40% having relapse within the treatment phase. Animals who relapse appear to have a more protracted course of signs and treatment duration. There is no current way to predict who will relapse.

### 12. Inflammatory Bowel Disease.

Inflammatory bowel disease ("IBD") in the canine, feline, and other species can be defined clinically as a spectrum of gastrointestinal disorders associated with chronic inflammation of the stomach, intestine and/or colon. A diagnosis of IBD is suspected only if the clinical signs have persisted chronically, usually at least 3 weeks. Often clinicians will make a presumptive diagnosis of IBD based on chronicity, clinical signs, failure to respond to symptomatic treatment and failure to continue with diagnostics for various causes. For this reason, many cases of IBD have an unknown etiology; however, certain forms of IBD (i.e., histiocytic in the Boxer breed of canines) is thought to have a genetic influence and there is strong evidence to support an immune mediated etiology.

The pathology of IBD has been directed at the immune system. The exact immune mechanism responsible is still unclear but IBD is thought to be the loss of immunologic tolerance to the normal bacterial flora or food antigens, leading to abnormal T cell immune reactivity in the gut microenvironment. Genetically engineered animal models that develop IBD involve alterations of T cell function suggesting that T cell populations are responsible for intestinal mucosal homeostatic regulation of immune responses. Immunohistochemical studies have shown an increase in the T cell population of the lamina propria, including CD3+ cells, CD4+ cells, as well as macrophages, neutrophils and Ig-A containing plasma cells. Many of the immunologic features of canine IBD can be explained by mucosal T cell activation. Enterocytes also play a role in the immunopathogenesis by acting like antigen presenting cells. Enterocytes also produce interleukins (IL-7, IL-15) during inflammation and activate mucosal lymphocytes. Therefore, a subset of CD4+ T cells within the intestinal epithelium that overproduce inflammatory cytokines with a concurrent loss of another subset of CD4+ T cells, and their associated cytokines, which normally regulate the inflammatory response and protect the gut from injury; as well as enterocytes acting as antigen presenting cells, all contribute to the pathogenesis of IBD,

### 13. Feline Cholangitis.

Feline cholangitis is the second most common liver disease in cats after hepatic lipidosis. Three forms of cholangitis have been recognized in cats: neutrophilic (bacterial or rarely protozoal), lymphocytic (immune mediated) and chronic (associated with liver fluke infection).

### 14. Feline Eosinophilic Disease.

Feline eosinophilic disease is a broad term that encompasses several eosinophilic reactions/granulomatous. Synonyms include feline eosinophilic granuloma complex, feline indolent ulcer, rodent ulcer, eosinophilic ulcer, eosinophilic plaque, feline linear granuloma and feline collagenolitic granuloma. The underlying allergic disease appears to provoke an episode although it is thought to have a genetic predisposition as well. Clinical signs include raised, well-demarcated erythematous yellow-to-pink colored linear to circular plaques are found. These lesions may be located on the ventrum, thighs, footpads, lip margins or chin. The lesions may also become ulcerated, are usually very pruritic and extremely painful.

### 15. Heart Disease.

Feline heart disease occurs in different forms with the most common being hypertrophic cardiomyopathy ("HCM") although processes such as myocarditis and/or infarction can occur with any type of heart disease. Secondary heart disease can also occur due to hyperthyroidism or hypertension. HCM occurs due to the combination of impaired ventricular relaxation and increased ventricular stiffness that leads to diastolic dysfunction. Most cats will also have dynamic outflow obstruction which causes the mitral valve to prolapse during systole and regurgitation occurs. This in turn eventually leads to increased atrial pressures then to congestive heart failure ("CHF"). HCM is most likely genetic in origin. Mutations in myosin binding protein C have been identified in Maine coons and Ragdolls with HCM, but gene mutation testing ("MBPC") is only available for the Maine coon.

### 16. Exercise-induced Pulmonary Hemorrhage.

("EIPH") is most often seen in race horses and other horses used for sport that undergo strenuous exercise for short periods of time. Epistaxis is observed only in approximately 5% of horses who are known to have exercise-induced pulmonary hemorrhage. Although it is thought all performance horses experience EIPH to some extent when exposed to strenuous activity, it rarely results in death, but does cause decrease lung function over time. Bleeding is caused by rupture of the pulmonary capillaries with subsequent pulmonary inflammation, fibrosis and angiogenesis which leads to further bleeding. Proposed mechanisms of the initial cause of EIPH include high pulmonary vascular pressures during maximum exercise, intrathoracic shear forces generated during exercise, failure of the pulmonary system to compensate or keep up with the extreme increase in cardiac output to meet the demand of high intensity exercise, coagulation dysfunction and/or neovascularization secondary to pulmonary inflammation. Currently, it is thought that with chronicity of disease, scarring of the lung will occur that will cause reduced gas exchange and therefore reduced athletic potential. Clinical signs include epistaxis, blood in the trachea after exercise, coughing, increased swallowing and/or prolonged recovery after exercise.

### 17. Exertional Rhabdomyolysis.

Exertional rhabdomyolysis ("ER", also known as tying up) can affect any horse but is a common disease of performance animals and can be a recurrent problem. There are different degrees of the disease to which a horse can be affected from subclinical to life threatening and commonly the time of onset after exercise correlates with severity of disease, with the earlier onset relating to more significant disease. ER occurs in response to an inadequate blood flow to the skeletal muscles of an exercising horse, with the lack of oxygenated blood, the muscle cells begin to function anaerobically to produce the needed ATP. The longer the horse exercises and/or the more predisposing factors present, the more muscle fibers that become damaged then the more severe the disease becomes. The more muscle cells/fibers involved then the more clinical signs that are seen. Eventually the muscle cell membrane breaks down, enzymes and myoglobin leak out which is then filtered by the renal system, hence the myoglobinuria and renal tubular damage that occurs with the life threatening form of the disease

An inherited and acquired form of the disease can occur. An animal with the inherited form is most likely to continue having recurrent episodes of ER. Inherited causes of ER originate from defective calcium regulation which is common in Thoroughbreds and causes a recurrent form of the disease. Polysaccharide storage myopathy ("PSSM") is a comparable inheritable myopathy that is often associated with ER and occurs commonly in quarter horses as well as other breeds. Acquired causes of the disease are many and usually occur as a combination etiology involving a horse undergoing unaccustomed exercise in addition to another predisposing factor such as: overfeeding carbohydrates (grain, pellets), sudden increase in work load in an animal with poor body condition, existing electrolyte or mineral imbalances (especially potassium), a deficiency in selenium or vitamin E (selenium levels should be measured before supplementation), hormone imbalance especially in fillies/mares, hypothyroidism, weather conditions being wet or cold. Females are more predisposed than males

### 18. Spinal Disease.

Spinal disease in rabbits, particularly in the aged rabbit, is not well defined or understood at this time. It is known that older rabbits can suffer from spinal disease that can be progressive and leave them in a paraplegic or ataxic state in severe cases. Clinical signs can be difficult to diagnose since rabbits do not readily show signs of pain. Veterinarians and owners must be astute in looking for the secondary signs of spinal disease and/or pain. Abnormalities of gait or unwillingness to move about are often overlooked if the rabbit spends most of its time confined to a hutch, rabbits may become more aggressive to owners or cage mates, they may have problems grooming themselves and therefore develop a soiled perineum area, perineal urine scald and associated dermatitis, inability to groom also leads to the buildup of scale therefore development of *Cheyletiella* is common, the rabbit usually can no longer reach the anus to ingest cecotrophs therefore secondary digestive disorders or hypomotility may occur, the rabbit may be less interactive than usual, ataxia, loss of conscious propriorception, urinary or fecal incontinence. Vertebral spondylosis, kyphosis or lordosis is a common finding in pet rabbits on radiographs and can cause pain, stiffness and degenerative disease. These spinal deformities can have many causes including congenital, a low calcium diet, metabolic bone disease, vitamin D deficiency, inactivity and/or a small cage size. Degenerative disc disease is disc protrusion and nuclear extrusion has been confirmed post-mortem as a cause of hind limb paralysis. This can be from forceful movement producing hyperflexion of the spine that may not result in a dislocation or fracture but may result in a disc lesion. Spontaneous degenerative spinal disease has also been studied in laboratory rabbits. These consist of chondroid metaplasia of the nucleus pulposus, calcification of the nucleus pulposus and spondylosis.

### 19. Myocardial Infarction.

Over 1.1 million Americans have a heart attack (myocardial infarction or "MI") each year. Although 80% survive the initial heart attack, nearly half become disabled with heart failure over the next six years. A heart attack occurs when a coronary artery becomes completely blocked so starving a section of heart muscle (myocardium) of oxygen and nutrients. If the blockage remains the section of heart muscle will die. The major complication in survivors is that in the days after the attack, tissues surrounding the dead zone are inadequately irrigated by collateral blood vessels, and also die off. The loss of heart muscle subsequently leads to the onset of heart failure. Enhanced blood flow to surviving heart muscle and, ultimately cardiac regeneration, is the essential goal in ensuring that risk of heart failure after heart attack is minimized.

### 20. Congestive Heart Failure.

Congestive heart failure ("CHF") typically occurs when an injured heart muscle is unable to pump strongly enough to maintain sufficient blood circulation to meet the needs of the body's other organs. Patients are constantly tired, short of breath, and in and out of hospital. One-third of patients with CHF require repeat hospitalization within three months after discharge.

### 21. Spinal Cord Injury

Spinal Cord Injury (SCI) is damage to the spinal cord that results in a loss of function such as mobility or feeling. Frequetit causes of damage are trauma (car accident, gunshot, falls, etc.) or disease (polio, spina bifida, Friedreich's Ataxia, etc.). The spinal cord does not have to be severed in order for a loss of functioning to occur. In fact, in most people with SCI, the spinal cord is intact, but the damage to it results in loss of functioning.

The effects of SCI depend on the type of injury and the level of the injury. SCI can be divided into two types of injury - complete and incomplete. A complete injury means that there is no function below the level of the injury; no sensation and no voluntary movement. Both sides of the body are equally affected. An incomplete injury means that there is some functioning below the primary level of the injury. A patient with an incomplete injury may be able to move one limb more than another, may be able to feel parts of the body that cannot be moved, or may have more functioning on one side of the body than the other. With the advances in acute treatment of SCI, incomplete injuries are becoming more common.

Besides a loss of sensation or motor functioning, individuals with SCI also experience other changes. For example, they may experience dysfunction of the bowel and bladder. Very high injuries (C-1, C-2) can result in a loss of many involuntary functions including the ability to breathe, necessitating breathing aids such as mechanical ventilators or diaphragmatic pacemakers. Other effects of SCI may include low blood pressure, inability to regulate blood pressure effectively, reduced control of body temperature, inability to sweat below the level of injury, and chronic pain.

### 22. Skeletal Muscle Fibrosis.

Skeletal muscle fibrosis is a life changing problem in individuals who suffer from disorders that target these muscles (Muscular Dystrophy, Multiple Sclerosis) or denervation atrophy induced by trauma or neuromuscular disease. Skeletal muscle fibrosis affects individuals of all race and ages including those with specific disease that suffer denervation fibrosis and those healthy athletes who over train or suffer a severe muscle injury. Microscopic tears that occur in musculature over time during exertion can cause muscle stiffness and fibrosis later in life that can become painful and even crippling.

### 23. Muscular Dystrophy.

Muscular dystrophy ("MD") refers to a group of hereditary muscle diseases that weakens the muscles that move the human body. Muscular dystrophies are characterized by progressive skeletal muscle weakness, defects in muscle proteins, and the death of muscle cells and tissue. Nine diseases including Duchene, Becker, limb girdle, congenital, scioscapulohumeral, myotonic, octilopharyiigeal, distal, and Emery-Dreifuss are classified as muscular dystrophy, although there are more than 100 diseases in total with similarities to muscular dystrophy. Most types of MD are multi-system disorders with manifestations in body systems including the heart, gastrointestinal and nervous systems, endocrine glands, skin, eyes and even brain. The condition may also lead to mood swings and learning difficulties.

### 24. Multiple Sclerosis.

Multiple sclerosis ("MS") is an autoimmune disease that affects the central nervous system. MS is caused by damage to the myelin sheath, the protective covering that surrounds nerve cells, it is a demyelinating disease. When this nerve covering is damaged, nerve impulses are slowed down or stopped. The nerve damage is caused by inflammation. Inflammation occurs when the body's own immune cells attack the nervous system. Repeated episodes of inflammation can occur along any area of the brain, optic nerve, and spinal cord. Because nerves in any part of the brain or spinal cord may be damaged, patients with multiple sclerosis can have symptoms in many different organ systems. Clinical symptoms can be generalized but are usually multiple. Symptoms of MS may mimic those of many other nervous system disorders.

### D. Clinical Results of Treatment.

### 1. Intervertebral Disc Disease, Spinal Cord Injury

According to one exemplary embodiment, a canine spinal injury patient exhibiting IVDD from an acute spinal injury incurred one month pre-treatment was treated with a therapeutic dose of MSCs derived from dental tissue and testicle tissue. Prior to the treatment regime, the patient displayed hind limb ataxia with a grade 3.5/5, and the patient could not support weight to walk up and down stairs. Further, the patient could not support weight long enough to walk a significant distance, had severe crepitus in distal thoracic and cranial lumbar spine, and could not run at all. Prior steroid and non-steroidal therapy was not helping at the time of treatment, although the patient was displaying normal bladder and bowel control.

The patient received three MSC intravenous injections at two week intervals, with one dose comprising MSCs derived from testicle tissue, and the last two injections derived from dental tissue. After receiving the treatment, the patient had a much improved hind limb ataxia to grade 1/5, was easily moving up and down stairways, was able to walk more than one mile daily, and was able to run with no administration of steroid or non-steroidal therapy. In fact, the very mild (grade 1) ataxia post-treatment was noticed only when the patient was very tired or over worked.

The following instances are not according to the invention and are present for illustration purposes only.

Yet another canine patient exhibiting chronic IVDD from to genetic hemi-vertebrae was treated with a therapeutic dose of dental tissue derived MSCs at one month intervals. Prior to treatment, this patient was utilizing a pull-cart for mobility, as no hind leg movement had been exhibited for approximately 1 year prior to treatment. Likewise, the patient had has developed arthritis in the front left elbow for the prior 6 months, making it difficult to use the cart effectively. The patient received three therapeutic doses of MSCs via intravenous injection at one month intervals. After the therapy, the patient developed some movement in the hind limbs, and mimics a "walking action" while utilizing the pull-cart, but is not yet able to support full body weight without use of the cart. The arthritis in the front elbow improved considerably, allowing greatly improved mobility with the cart.

According to another instance, a lagomorph patient exhibiting chronic spinal disease (IVDD) leading to paresis of hind limbs was treated with a single therapeutic dose of bone marrow stem cells administered intravenously. Prior to treatment, the patient had become non-ambulatory for several months, despite being bright and alert with a good appetite prior to the onset of the disease. Prior to treatment with a therapeutic dose of bone marrow derived MSCs, the patient was being treated daily through physical therapy, but the patient began to not be able to move her front legs or lift her head/neck region and showed signs of depression one month prior to presentation. After intravenous treatment with a therapeutic dose of MSCs, the patient became bright and alert again, began eating better and began trying to move her front limbs and head. The patient was able to start lifting her head/neck region and front arms similar to the extent shown prior to the onset of the disease.

### 2. Chronic Osteoarthritis.

Yet another canine patient was treated for chronic OA with a single therapeutic dose of dental tissue derived MSCs. Prior to treatment, this patient was taking long term (approximately 1.5 years) non-steroidal anti-inflammatory medications which required frequent blood work to monitor liver and kidney function. After treatment, the patient's energy and hair coat improved, and the patient was able to cease all OA medications. The patient's activity greatly improved, and the beneficial results were maintained until another therapeutic dose of MSCs was required approximately 6 months after the treatment.

According to another instance a geriatric feline was treated for chronic osteoarthritis with a single therapeutic dose of dental tissue derived MSCs. Prior to treatment, the patient's chronic osteoarthritis had developed to a level that significantly affected activity level, and had significantly affected the gross anatomic structure of the patient's front limbs at the elbow level. However, the patient was on no medications prior to the treatment. After treatment, the patient displayed increased activity, reduced lethargy and sleep time, and showed a marked improvement in appetite.

According to another instance, a geriatric lagomorph patient with severe generalized arthritis, primarily of the spine and all limbs, was treated with a single therapeutic dose of bone marrow derived MSCs. Prior to treatment, the patient spend the majority of its day with little movement, was on pain medication and non-steroidal anti-inflammatory medication to treat the osteoarthritis, and was prone to self-soiling. After the single treatment, the patient became extremely active, moving around on his own. Further, the patient was again able to posture to urinate and defecate, decreasing the prior problem of self-soiling. The patient's overall health, appetite, and body weight increased, allowing the anti-inflammatory medications to be discontinued.

According to another instance, a geriatric lagomorph patient with severe generalized arthritis, especially of the front limbs due to a previous front limb fracture and subsequent surgery of the area, was treated with a single therapeutic dose of bone marrow derived MSCs. Prior to the treatment, the patient's previously fractured front limb was significantly deformed, causing the patient to fall often. Further, the patient was quite sedentary, was taking a pain medication, a glucosamine-chondroitin supplement and a non-steroidal anti-inflammatory. After the single treatment, the patient was able to discontinue the pain medication, and was more ambulatory and interactive with an increased appetite.

### 3. Inflammatory Bowel Disease (IBD).

Another canine patient was treated for chronic OA with a two therapeutic doses of dental tissue derived MSCs spaced approximately three months apart. Prior to the treatment, the patient was on a prescription diet, and was administered several different medications twice daily to help control severe lymphocytic-plasmacytic colitis. As the disease progressed it was more difficult to control with medication/diet and the patient began losing significant amounts of weight, with the patient's hair becoming dry and brittle, signifying poor uptake of nutrients.

After the treatment, the patient remained on the pre-treatment medication, but after the first therapeutic dose of MSCs was administered, the patient's feces changed from a watery, bloody stool to a semi-formed, non-hemorrhagic stool. The patient regained ¾ of the weight lost prior to the treatment, and the patient's hair improved dramatically. Thereafter, two of the medications for treatment of lymphocytic-plasmacytic colitis were discontinued altogether, with all other treatments remaining unchanged. The patient became more active and displayed better overall health.

### 4. Early stage Degenerative Myelopathy (DM).

Another canine patient was treated for degenerative myelopathy with a single therapeutic dose of dental derived MSCs given intravenously. Prior to treatment, the patient was becoming ataxic in the hind limbs with conscious proprioception deficits. The patient would "sway" when walking, and had difficulty negotiating stairs and moving around the house. Post therapy, the patient displayed increased strength and vigor overall, and had notable ataxia improvement with no noticeable digression for approximately four months after treatment.

### 5. Cardiomyopathy.

According to another instance another feline patient was treated for a generalized inflammatory disorder of unknown etiology and feline cardiomyopathy diagnosed via echocardiography with a single therapeutic dose of dental derived MSCs given intravenously. Prior to treatment, the patient's condition was not stabilized the patient was displaying marked lethargy. After treatment, echocardiography measurements showed noticeable improvement of the cardiomyopathy symptoms. Further, the patient showed dramatic improvement of the general lethargy noted pre-treatment, and showed an activity and appetite associated with a normal individual.

### 6. Muscle Fibrosis.

According to another instance, a lagomorph patient was treated for IVDD and muscle fibrosis of the hind limbs with a single dose of bone marrow derived MSCs intravenously. Prior to treatment, the patient displayed chronic spinal disease leading to paresis of hind limbs and eventually fibrosis of musculature of affected limbs. While the front limbs did not show signs of muscular fibrosis, deformation of the front limbs was noted due to overuse. The patient was utilizing a mobility cart for 6 months prior to treatment.

After treatment, the patient's hind limb fibrosis greatly improved to the point that the leg could be flexed and manipulated for physical therapy to occur, and the patient's hind limbs were no longer frozen in one position. As physical therapy continued, the patient began to regain the ability to bend her legs underneath her under her own power, and was able to balance herself without her cart and take some steps on her own. The patient displayed improved overall general health.

According to another instance, an equine patient was treated for long term muscle fibrosis and OA from previous right scapular fracture with one therapeutic dose of placental derived MSCs administered three times at monthly intervals. Prior to treatment, the patient's front right shoulder showed severe supraspinatus and infraspinatus muscle fibrosis, lack of blood supply to the site according to a thermography study, decreased range of motion in the affected limb as well as atrophy of all shoulder and proximal arm musculature. Osteoarthritis of the shoulder became a problem for the arm as a sequential disease to the trauma as well. After the treatments, the patient's muscle fibrosis greatly improved, and blood supply to the area greatly improved as shown by thermography comparison studies. Further, the patient's range of motion in the affected limb improved dramatically, the shoulder muscle mass as measured by the circumference of the leg measurably improved from previous records, and the patient was able to discontinue prior OA medication.

### 7. Exercise Induced Pulmonary Hemorrhage (EIPH).

According to one instance, an equine patient diagnosed with EIPH via endoscopy and bronchoalveolar lavage ("BAL") was treated with four therapeutic doses of placental derived MSCs administered intravenously, with each dose administered at monthly intervals. Prior to treatment, the patient would tire easily and failed to finish well in races. Further, the patient displayed a thin overall body condition score, measuring a 2/5. After treatment, a post-race endoscopy and BAL testing showed marked improvement in the pulmonary hemorrhage (EIPH), the patient was not winded after racing, and consistently placed in finishing positions in races. Further, the patient's body condition score improved to 3.5/5.

### 8. Rhabdomyolysis.

An equine patient diagnosed with exercised induced rhabdomyolysis, severe generalized muscle soreness with associated electrolyte abnormalities, was treated with one therapeutic dose of dental derived MSCs intravenously. Prior to treatment, clinical signs of exercised induced rhabdomyolysis persisted for days after and episode of extreme exercise. After treatment, the patient was able to move around normally, eat normally and begin exercise regimen. Further, the patient showed diminished signs of pain in normal stance and gait.

### 9. Spinal Cord Injury.

An equine patient displaying a caudal spinal cord injury from an impact with a wall during a race was treated with four therapeutic doses of dental derived MSC, with each dose administered at monthly intervals. Prior to the treatment, the patient was ataxic in the hind limbs and without control of urinary, rectal, or penile function. Initial emergency treatment improved the hind limb ataxia to a mild ataxia, allowing the patient to walk/trot. However the patient required catheterization in order to relieve his bladder and avoid bladder rupture, which occurred in one instance. After treatment, the patient regained control of his rectal function and further regained enough control of penile function to allow at least partial re-sheathing of the member to prevent damage from drying out. Further, the patient regained the ability to urinate without being catheterized.

## Claims

1. A mesenchymal stem cell composition for use in treating in a canine or feline patient a preselected disease or diseased state selected from:
degenerative bone disease, osteoarthritis, rheumatoid arthritis, polyarthritis, systemic lupus erythematosus, inflammatory bowel disease, atopy, hepatitis, chronic steroid responsive meningitis-arteritis, beagle pain syndrome, chronic renal failure disease, keratoconjunctivitis sicca, immune mediated non-erosive arthritis, immune mediated hemolytic anemia, immune mediated thrombocytopenia, Evans syndrome, intervertebral disc disease, refractory corneal ulcer, diabetes mellitus, eosinophilic granuloma complex, cholangitis, exercise induced pulmonary hemorrhage, rhabdomyolysis, corneal ulcer, eczema, multiple sclerosis, muscular dystrophy, and muscle fibrosis secondary to disease or trauma,
wherein the treatment comprises the step of systemically administering a therapeutic dose of a mesenchymal stem cell composition through an intravenous injection,
the mesenchymal stem cell composition comprising mesenchymal stem cells harvested from at least one tissue selected from the group consisting of testicle tissue and uterine tissue, wherein the mesenchymal stem cell composition comprises a solution having a concentration of approximately 2,000,000 cells or less per mL of solution, wherein the mesenchymal stem cells are allogeneic or autologous to the patient.

2. A mesenchymal stem cell composition for use in treating in a canine or feline patient
a preselected disease or diseased state,
wherein the treatment consists of the step of systemically administering a therapeutic dose of a mesenchymal stem cell composition through an intravenous injection,
the mesenchymal stem cell composition comprising mesenchymal stem cells harvested from at least one tissue selected from the group consisting of testicle tissue and uterine tissue, wherein the mesenchymal stem cell composition comprises a solution having a concentration of approximately 2,000,000 cells or less per mL of solution, and wherein the mesenchymal stem cells are allogeneic or autologous to the patient, and
wherein the preselected disease or diseased state is selected from the group consisting of: degenerative bone disease, osteoarthritis, rheumatoid arthritis, polyarthritis, systemic lupus erythematosus, inflammatory bowel disease, atopy, hepatitis, chronic steroid responsive meningitis-arteritis, beagle pain syndrome, degenerative myelopathy, chronic renal failure disease, keratoconjunctivitis sicca, immune mediated non-erosive arthritis, immune mediated hemolytic anemia, immune mediated thrombocytopenia, Evans syndrome, intervertebral disc disease, refractory corneal ulcer, diabetes mellitus, spinal trauma, eosinophilic granuloma complex, cholangitis, exercise induced pulmonary hemorrhage, rhabdomyolysis, corneal ulcer, eczema, multiple sclerosis, muscular dystrophy, spinal injury, and muscle fibrosis secondary to disease or trauma.

3. The mesenchymal stem cell composition for use according to claim 1 or claim 2, wherein the therapeutically effective dose is about 6 million mesenchymal stem cells per kg of a patient's body weight.

4. The mesenchymal stem cell composition for use according to claim 3, wherein the therapeutically effective dose does not exceed about 50 million mesenchymal stem cells regardless of the patient's body weight.

5. The mesenchymal stem cell composition for use according to claim 1 or claim 2, wherein the mesenchymal stem cell composition consists essentially of mesenchymal stem cells and saline.

6. The mesenchymal stem cell composition for use according to claim 1 or claim 2, wherein the mesenchymal stem cell composition includes mesenchymal stem cells and saline at a concentration of no more than 500,000 cells per mL.

7. The mesenchymal stem cell composition for use according to claim 6, wherein the mesenchymal stem cell composition includes mesenchymal stem cells and saline at a concentration of no more than 100,000 cells per mL.

8. The mesenchymal stem cell composition for use according to claim 7, wherein the mesenchymal stem cell composition further comprises factors from a stem cell conditioned media.

## Patentansprüche

1. Eine mesenchymale Stammzellzusammensetzung zur Verwendung bei einem Hunde- oder Katzenpatienten zum Behandeln einer zuvor ausgewählten Erkrankung oder eines zuvor ausgewählten Krankheitszustands, ausgewählt aus: degenerativer Knochenerkrankung, Arthrose, rheumatoider Arthritis, Polyarthritis, systemischem Lupus erythematodes, entzündlichen Darmerkrankungen, Atopie, Hepatitis, chronischer auf Steroide ansprechender Meningitis-Arteriitis, Beagle-Schmerzsyndrom, chronischem Nierenversagen, Keratoconjunctivitis sicca, immunvermittelter nicht-erosiver Arthritis, immunvermittelter hämolytischer Anämie, immunvermittelter Thrombozytopenie, Evans-Syndrom, Bandscheibenerkrankung, refraktärem Korneaulkus, Diabetes mellitus, eosinophilem Granulomkomplex, Cholangitis, belastungsinduzierter Lungenblutung, Rhabdomyolyse, Korneaulkus, Ekzem, multipler Sklerose, Muskeldystrophie und Muskelfibrose im Anschluss an eine Erkrankung oder ein Trauma,
wobei die Behandlung den Schritt des systemischen Verabreichens einer therapeutischen Dosis einer mesenchymalen Stammzellzusammensetzung durch eine intravenöse Injektion beinhaltet,
wobei die mesenchymale Stammzellzusammensetzung mesenchymale Stammzellen beinhaltet, die von mindestens einem Gewebe geerntet wurden, das aus der Gruppe ausgewählt ist, die aus Hodengewebe und Uterusgewebe besteht, wobei die mesenchymale Stammzellzusammensetzung eine Lösung mit einer Konzentration von ungefähr 2,000,000 Zellen oder weniger pro ml Lösung beinhaltet, wobei die mesenchymalen Stammzellen allogen oder autolog für den Patienten sind.

2. Mesenchymale Stammzellzusammensetzung zur Verwendung bei einem Hunde- oder Katzenpatienten zum Behandeln einer zuvor ausgewählten Erkrankung oder eines zuvor ausgewählten Krankheitszustands,
wobei die Behandlung aus dem Schritt des systemischen Verabreichens einer therapeutischen Dosis einer mesenchymalen Stammzellzusammensetzung durch eine intravenöse Injektion besteht,
wobei die mesenchymale Stammzellzusammensetzung mesenchymale Stammzellen, die von mindestens einem Gewebe geerntet wurden, das aus der Gruppe ausgewählt ist, die aus Hodengewebe und Uterusgewebe besteht, wobei die mesenchymale Stammzellzusammensetzung eine Lösung mit einer Konzentration von ungefähr 2,000,000 Zellen oder weniger pro ml Lösung beinhaltet, und wobei die mesenchymalen Stammzellen allogen oder autolog für den Patienten sind und
wobei die zuvor ausgewählte Erkrankung oder der zuvor ausgewählte Krankheitszustand aus der Gruppe ausgewählt ist, die aus Folgenden besteht: degenerativer Knochenerkrankung, Arthrose, rheumatoider Arthritis, Polyarthritis, systemischem Lupus erythematodes, entzündlicher Darmerkrankung, Atopie, Hepatitis, chronischer auf Steroide ansprechender Meningitis-Arteriitis, Beagle-Schmerzsyndrom, degenerativer Myelopathie, chronischem Nierenversagen, Keratoconjunctivitis sicca, immunvermittelter nicht-erosiver Arthritis, immunvermittelter hämolytischer Anämie, immunvermittelter Thrombozytopenie, Evans-Syndrom, Bandscheibenerkrankung, refraktärem Korneaulkus, Diabetes mellitus, Wirbelsäulentrauma, eosinophilem Granulomkomplex, Cholangitis, belastungsinduzierter Lungenblutung, Rhabdomyolyse, Korneaulkus, Ekzem, multipler Sklerose, Muskeldystrophie, Rückenmarksverletzung und Muskelfibrose im Anschluss an eine Erkrankung oder ein Trauma.

3. Mesenchymale Stammzellzusammensetzung zur Verwendung nach Anspruch 1 oder Anspruch 2, wobei die therapeutisch wirksame Dosis etwa 6 Millionen mesenchymale Stammzellen pro kg Körpergewicht eines Patienten beträgt.

4. Mesenchymale Stammzellzusammensetzung zur Verwendung nach Anspruch 3, wobei die therapeutisch wirksame Dosis unabhängig von dem Körpergewicht des Patienten etwa 50 Millionen mesenchymale Stammzellen nicht überschreitet.

5. Mesenchymale Stammzellzusammensetzung zur Verwendung nach Anspruch 1 oder Anspruch 2, wobei die mesenchymale Stammzellzusammensetzung im Wesentlichen aus mesenchymalen Stammzellen und Kochsalzlösung besteht.

6. Mesenchymale Stammzellzusammensetzung zur Verwendung nach Anspruch 1 oder Anspruch 2, wobei die mesenchymale Stammzellzusammensetzung mesenchymale Stammzellen und Kochsalzlösung mit einer Konzentration von nicht mehr als 500,000 Zellen pro ml umfasst.

7. Mesenchymale Stammzellzusammensetzung zur Verwendung nach Anspruch 6, wobei die mesenchymale Stammzellzusammensetzung mesenchymale Stammzellen und Kochsalzlösung mit einer Konzentration von nicht mehr als 100,000 Zellen pro ml umfasst.

8. Mesenchymale Stammzellzusammensetzung zur Verwendung nach Anspruch 7, wobei die mesenchymale Stammzellzusammensetzung ferner Faktoren aus einem stammzellkonditionierten Medium umfasst.

## Revendications

1. Composition de cellules souches mésenchymateuses destinée à une utilisation dans le traitement chez un patient canin ou félin d'une maladie ou d'un état pathologique présélectionné(e) qui est sélectionné(e) parmi une maladie dégénérative osseuse, une arthrose, une polyarthrite rhumatoïde, une polyarthrite, un lupus érythémateux disséminé, une maladie inflammatoire de l'intestin, une atopie, une hépatite, une méningite-artérite chronique sensible aux stéroïdes, un syndrome douloureux du beagle, une maladie d'insuffisance rénale chronique, une kératoconjonctivite sèche, une arthrite non érosive à médiation immunitaire, une anémie hémolytique à médiation immunitaire, une thrombocytopénie à médiation immunitaire, un syndrome d'Evans, une maladie du disque intervertébral, un ulcère cornéen réfractaire, un diabète sucré, un complexe granulome éosinophilique, une angiocholite, une hémorragie pulmonaire induite par l'exercice, une rhabdomyolyse, un ulcère cornéen, un eczéma, une sclérose en plaques, une dystrophie musculaire et une fibrose musculaire secondaire à une maladie ou à un traumatisme,
le traitement comprenant l'étape qui consiste en une administration systémique d'une dose thérapeutique d'une composition de cellules souches mésenchymateuses par injection intraveineuse,
la composition de cellules souches mésenchymateuses comprenant des cellules souches mésenchymateuses prélevées dans au moins un tissu sélectionné dans le groupe constitué d'un tissu testiculaire et d'un tissu utérin, la composition de cellules souches mésenchymateuses comprenant une solution ayant une concentration d'environ 2,000,000 cellules ou moins par ml de solution, les cellules souches mésenchymateuses étant allogéniques ou autologues pour le patient.

2. Composition de cellules souches mésenchymateuses destinée à une utilisation dans le traitement chez un patient canin ou félin d'une maladie ou d'un état pathologique présélectionné(e),
le traitement se composant de l'étape qui consiste en une administration systémique d'une dose thérapeutique d'une composition de cellules souches mésenchymateuses par injection intraveineuse, la composition de cellules souches mésenchymateuses comprenant des cellules souches mésenchymateuses prélevées dans au moins un tissu sélectionné dans le groupe constitué d'un tissu testiculaire et d'un tissu utérin, la composition de cellules souches mésenchymateuses comprenant une solution ayant une concentration d'environ 2,000,000 cellules ou moins par ml de solution, et les cellules souches mésenchymateuses étant allogéniques ou autologues pour le patient, et
la maladie ou l'état pathologique présélectionné(e) étant sélectionné(e) dans le groupe constitué d'une maladie dégénérative osseuse, d'une arthrose, d'une polyarthrite rhumatoïde, d'une polyarthrite, d'un lupus érythémateux disséminé, d'une maladie inflammatoire de l'intestin, d'une atopie, d'une hépatite, d'une méningite-artérite chronique sensible aux stéroïdes, d'un syndrome douloureux du beagle, d'une myélopathie dégénérative, d'une maladie d'insuffisance rénale chronique, d'une kératoconjonctivite sèche, d'une arthrite non érosive à médiation immunitaire, d'une anémie hémolytique à médiation immunitaire, d'une thrombocytopénie à médiation immunitaire, d'un syndrome d'Evans, d'une maladie du disque intervertébral, d'un ulcère cornéen réfractaire, d'un diabète sucré, d'un traumatisme rachidien, d'un complexe granulome éosinophilique, d'une angiocholite, d'une hémorragie pulmonaire induite par l'exercice, d'une rhabdomyolyse, d'un ulcère cornéen, d'un eczéma, d'une sclérose en plaques, d'une dystrophie musculaire, d'une lésion de la colonne vertébrale et d'une fibrose musculaire secondaire à une maladie ou à un traumatisme.

3. Composition de cellules souches mésenchymateuses destinée à une utilisation selon la revendication 1 ou la revendication 2, la dose thérapeutiquement efficace étant d'environ 6 millions de cellules souches mésenchymateuses par kg de poids corporel d'un patient.

4. Composition de cellules souches mésenchymateuses destinée à une utilisation selon la revendication 3, la dose thérapeutiquement efficace ne dépassant pas environ 50 millions de cellules souches mésenchymateuses quel que soit le poids corporel du patient.

5. Composition de cellules souches mésenchymateuses destinée à une utilisation selon la revendication 1 ou la revendication 2, la composition de cellules souches mésenchymateuses se composant essentiellement de cellules souches mésenchymateuses et d'une solution saline.

6. Composition de cellules souches mésenchymateuses destinée à une utilisation selon la revendication 1 ou la revendication 2, la composition de cellules souches mésenchymateuses comprenant des cellules souches mésenchymateuses et une solution saline à une concentration ne dépassant pas 500,000 cellules par ml.

7. Composition de cellules souches mésenchymateuses destinée à une utilisation selon la revendication 6, la composition de cellules souches mésenchymateuses contenant des cellules souches mésenchymateuses et une solution saline à une concentration ne dépassant pas 100,000 cellules par ml.

8. Composition de cellules souches mésenchymateuses destinée à une utilisation selon la revendication 7, la composition de cellules souches mésenchymateuses comprenant en outre des facteurs provenant d'un milieu conditionné par des cellules souches.
